Europäisches Patentamt

**European Patent Office**

**Office européen des brevets**

(19)

(11) Veröffentlichungsnummer: **0 004 925**
**B1**

(12) **EUROPÄISCHE PATENTSCHRIFT**

(45) Veröffentlichungstag der Patentschrift: **24.02.82**

(21) Anmeldenummer: **79101080.4**

(22) Anmeldetag: **09.04.79**

(51) Int. Cl.³: **A 61 K 31/545,**
**A 61 K 31/71 //(A61K31/545,**
**31/545), (A61K31/545,**
**31/43), (A61K31/71, 31/545)**

(54) **Synergistische Mischungen von Antibiotika und Verfahren zu ihrer Herstellung.**

(30) Priorität: **19.04.78 CH 4194/78**

(43) Veröffentlichungstag der Anmeldung:
**31.10.79 Patentblatt 79/22**

(45) Bekanntmachung des Hinweises auf die Patenterteilung:
**24.02.82 Patentblatt 82/8**

(84) Benannte Vertragsstaaten:
**BE CH DE FR GB IT LU NL SE**

(56) Entgegenhaltungen:
GB - A - 1 310 642

CHEMICAL ABSTRACTS, Vol. 70, Nr. 4,
27. Januar 1969,
Columbus, Ohio, USA,
R. P. MOUTON: "Cephalosporins in antibiotic
combinations in vitro"
Seite 1780, Zusammenfassung Nr. 17812j.

(73) Patentinhaber: **CIBA-GEIGY AG**
**Patentabteilung Postfach**
**CH-4002 Basel (CH)**

(72) Erfinder: **Kradolfer, Friedrich, Dr.**
**Brudeholzallee 202**
**CH-4059 Basel (CH)**
Erfinder: **Zak, Otokar, Dr.**
**Am Stausee 27/17**
**CH-4127 Birsfelden (CH)**

(56) Entgegenhaltungen:

**CHEMICAL ABSTRACTS, Vol. 85 Nr. 7,**
**16. August 1976,**
**Columbus, Ohio, USA,**
**L. D. SABATH: "Combinations of penicillins and**
**cephalosporins",**
**Seite 104, Zusammenfassung Nr. 41430p.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# 0 004 925

Synergistische Mischungen von Antibiotika und Verfahren zu ihrer Herstellung

Die vorliegende Erfindung betrifft neue synergistische antibiotische Mischungen von antibiotisch wirkenden Verbindungen mit gegenüber den Einzelkomponenten vorteilhaften Eigenschaften, pharmazeutische Präparate enthaltend diese Mischungen und Verfahren zu ihrer Herstellung, sowie die Verwendung dieser Präparate zur Bekämpfung von Infektionen.

Die erfindungsgemässen synergistischen Mischungen enthalten als aktive Komponenten das Antibiotikum Cefsulodin-Natrium und ein zweites Antibiotikum aus der Gruppe Clavulansäure, Penicillansäure-4,4-dioxid, Azlocillin, Mezlocillin, Piperacillin, Mecillinam, Cephacetril, Cefoxitin, Cefotiam, Amikacin, Dibekacin, Tobramycin, Sisomicin oder Gentamicin oder ein Salz davon.

Cefsulodin-Natrium ist die internationale Freibezeichnung (International Nonproprietary Name, INN) für das Natriumsalz von N-[7$\beta$-($\alpha$-Sulfophenylacetamido)-ceph-3-em-3-ylmethyl]-4'-carbamoyl-pyridinium-4-carboxylat. Die Verbindung ist aus der Deutschen Offenlegungsschrift 2.234.280 bekannt. Ihre antibiotische Aktivität richtet sich gegen Gram-negative Kokken, Grampositive Kokken und Bakterien und insbesondere gegen Pseudomonas Stämme, sowohl gegen Carbenicillin-sensitive als auch Carbencillin-resistente. Cefsulodin-Natrium hat eine relativ geringe Wirksamkeit gegen Enterobakterien, wie Escherichia coli und Klebsiella pneumoniae, und Anaerobier, z.B. Bacteroides.

Ueberraschenderweise wurde nun gefunden, dass sich die antibiotischen Wirkungen der genannten Antibiotika und diejenigen von Cefsulodin-Natrium in den erfindungsgemässen Mischungen in synergistischer Weise steigern und dass gegebenenfalls sogar eine Steigerung der Verträglichkeit gegenüber den Einzelkomponenten auftritt. Dieser Synergismus wurde überraschenderweise auch bei Enterobakterien, Anaerobiern und bei Mischinfektionen festgestellt.

Die Vorteile von synergistischen Mischungen gegenüber den Einzelkomponenten sind allgemein bekannt und können auf verschiedene Weise genutzt werden. Bei den vorliegenden Antibiotikamischungen liegen sie sowohl in einer Verringerung der Dosis bei gleichem antibiotischem Effekt, was insbesondere eine geringere Belastung des Organismus mit Fremdsubstanzen und damit geringere Nebenwirkungen bedeutet, als auch in der Verbreiterung des Wirkungsspektrums, was bei der Behandlung von Mischinfektionen, insbesondere von Begleitinfektionen bei Pseudomonas-Infektionen, von Wichtigkeit ist. Ausserdem ist eine verminderte Tendenz bei der Entwicklung resistenter Stämme zu erwarten. Durch geeignete Kombination können auch Stämme bekämpft werden, die von den einzelnen Partnern allein nicht beeinflusst werden.

Die für die synergistischen Mischungen geeigneten Antibiotika können, je nach ihren Lösungseigenschaften und abhängig vom Vorhandensein basischer und/oder saurer Gruppen, als freie Verbindungen oder als Salze eingesetzt werden.

Antibiotika mit sauren und basischen Gruppen können in Form von inneren Salzen, d.h. in zwitterionischer Form, eingesetzt werden. Antibiotika die überwiegend basischen Charakter besitzen, können als stabile Säureadditionssalze, z.B. mit anorganischen Säuren, wie Salzsäure, Schwefelsäure oder Phosphorsäure, oder mit geeigneten organischen Carbon- oder Sulfonsäuren, wie mit aliphatischen Mono-, Di- oder Tricarbonsäuren, z.B. Essigsäure, Malonsäure, Weinsäure, Zitronensäure, 4-(N,N-Dipropylsulphamoyl)-benzoesäure (Probenecid), mit p-Toluolsulfonsäure, $\alpha$- oder $\beta$-Naphthalinsulfonsäure oder Naphthalin-disulfonsäure, insbesondere Naphthalin-1,5-disulfonsäure, verwendet werden. Antibiotika, die überwiegen sauren Charakter besitzen, können stabile Salze mit Basen bilden und in dieser Form eingesetzt wurden. Bevorzugte Salze dieser Art sind insbesondere pharmazeutisch verwendbare, nicht-toxische Salze wie Alkalimetall-oder Erdalkalimetall-, z.B. Natrium-, Kalium-, Magnesium-oder Calciumsalze, ferner Ammoniumsalze mit Ammoniak oder geeigneten organischen Aminen, wobei in erster Linie aliphatische, cycloaliphatische, cycloaliphatisch-aliphatische und araliphatische, primäre, sekundäre oder tertiäre Mono-, Di- oder Polyamine, sowie heterocyclische Basen für die Salzbildung in Frage kommen, wie Niederalkylamine, z.B. Triäthylamin, Hydroxyniederalkylamine, z.B. 2-Hydroxyäthylamin, Bis-(2-hydroxyäthyl)-amin oder Tris-(2-hydroxyäthyl)-amin, basische aliphatische Ester von Carbonsäuren, z.B. 4-Aminobenzoesäure-(2-diäthylaminoäthyl)-ester, Niederalkylenamine, z.B. 1-Aethylpiperidin, Cycloalkylamine, z.B. Dicyclohexylamin, oder Benzylamine, z.B. N,N'-Dibenzyläthylendiamin, ferner Basen vom Pyridintyp, z.B. Pyridin, Collidin oder Chinolin.

In einer erfindungsgemässen synergistischen Mischung kann das Mischungsverhältnis von Cefsulodin-Natrium zum zweiten Antibiotikum in weiten Grenzen variieren.

Die Grenzmischungsverhältnisse, bei denen noch Synergismus auftritt, hängen vom Mikroorganismus, vom verwendeten Testsystem und natürlich vom zweiten Antibiotikum ab. Die Erfindung betrifft insbesondere synergistische Mischungen, worin die Gewichtsverhältnisse Cefsulodin-Natrium zum zweiten Antibiotikum unter Erhaltung des synergistischen Effektes zwiscen etwa 1:0,001 und 1:4000 liegen. Die Erfindung betrifft insbesondere Mischungen von Cefsulodin-Natrium (=1) mit Mecillinam in den Gewichtsverhältnissen 1:0,04 bis 1:128, mit Azlocillin, Piperacillin oder Mezlocillin in den Gewichtsverhältnissen 1:0,04 bis 1:3125, mit Amikacin, Dibekacin, Tobramycin, Sisomicin oder Gentamicin in den Gewichtsverhältnissen 1:0,004 bis 1:5, insbesondere 1:0,06 bis 1:0,09, und mit Cephace-

2

tril, Cefoxitin oder Cefotiam in den Gewichtsverhältnissen 1:0,0155 bis 1.5.

Die Erfindung betrifft insbesondere die in den Beispielen beschriebenen Mischungen.

Die synergistischen Mischungen der vorliegenden Erfindung werden zur Herstellung von pharmazeutischen Präparaten benutzt, welche eine wirksame Menge der oben genannten Wirkstoffgemische allein oder im Gemisch mit üblichen anorganischen oder organischen, festen oder flüssigen, pharmazeutisch verwendbaren Träger- oder Hilfsstoffen, z.B. Konservier-, Stabilisier-, Netz- und/oder Emulgiermittel, Löslichkeitsvermittler, Salze zur Regulierung des osmotischen Druckes und/oder Puffer enthalten. Solche Hilfsstoffe sind beispielsweise Kohlenhydrate, wie Saccharose, Lactose, Dextrose, Sukrose, Sorbitol, Cellulose oder Cellulosederivate, wie Methylcellulose, Natriumcarboxymethylcellulose, Polyäthylenglykol, Polyvinylpyrrolidon und insbesondere D-Mannit.

Die pharmazeutischen Präparate enthalten 0,1% bis 100%, insbesondere 1% bis 90% des Wirkstoffgemisches und sind zur bequemen Dosierung, gewöhnlich in Doseneinheitsformen von 0,1 g bis 10 g, beispielsweise in Ampullen oder Vials abgefüllt. Eine bevorzugte Form sind Trockenampullen aus denen vor Gebrauch eine Injektions-, Infusions-, oder Tropflösung durch Zugabe der benötigten Menge gegebenenfalls pyrogenfreien Wassers oder eines anderen physiologisch verträglichen Lösungsmittels, wie physiologischer Natriumchloridlösung oder eines Plasma enthaltende Lösungsmittels, hergestellt werden kann.

Die Herstellung der pharmazeutischen Präparate erfolgt auf an sich bekannte und übliche Weise, gewöhnlich auf nicht-chemischem Wege, z.B. mittels konventioneller Misch-, Lösungs-, Trocknungs- oder Lyophilisierungsverfahren.

Die synergistischen Gemische, bzw. die daraus herstellbaren pharmazeutischen Präparate, werden vor allem parenteral, insbesondere intravenös, intramuskulär oder subcutan verabreicht. Die Applikation erfolgt entweder einmalig in hohen Dosen (Stosstherapie), in kleineren aufeinanderfolgenden Dosen oder als Dauerapplikation (z.B. Dauerinfusion). Die Dosierung richtet sich nach der Art und dem Schweregrad der Infektion, dem Gewicht und dem Allgemeinzustand des Patienten und der Applikationsart und muss vom Arzt von Fall zu Fall bestimmt werden. Im allgemeinen liegt die Dosierung zwischen 1 und 100 mg/kg bei parenteraler Applikation.

Für die synergistischen Mischungen enthaltend Cefsulodin-Natrium und einem der genannten Aminoglycoside liegt die Dosierung bei etwa 3 bis 63 mg/kg, insbesondere bei etwa 3,5 bis 40 mg/kg, wobei die Mischungsverhältnisse Cefsulodin-Natrium zum Aminoglycosid, z.B. zu Amikacin, bevorzugt zwischen etwa 1:0,06 und 1:0,22 liegen. Für die Mischungen mit stärker wirksamen Vertretern aus der genannten Gruppe der Aminoglycoside, z.B. mit Gentamycin, liegt die Dosierung zwischen etwa 3,5 bis 36 mg/kg, und die Mischungsverhältnisse liegen zwischen etwa 1:0,06 und 1:0,09.

Der folgende experimentelle Teil und die Beispiele dienen zur Illustration der Erfindung.

Experimenteller Teil

Die synergistischen, d.h. überadditiven, antibiotischen Wirkungen wurden mittels der folgenden Versuchsanordnungen festgestellt:

1. Nachweis der synergistischen Wirkung *in vitro:*

Die zu kombinierenden Substanzen A und B werden in DST Broth OXOID in Schachbrettanordnung mit Hilfe von Microtitersystem in 1:2 Schritten verdünnt. Dabei werden alle Konzentrationen der Substanz A mit allen Konzentrationen der Substanz B kombiniert und anschliessend mit einer standardisierten Bakteriensuspension (Endkonzentration ca. $10^4$ Keime/ml) beimpft. Nach 18 Stunden Inkubation bei 37°C wird die Hemmung des Bakterienwachstums abgelesen. Die hemmenden Konzentrationen werden gemäss Kerry et al. (J. Antimicrob. Chemotherapy 1, 417—427, 1975) in "Fractional Inhibition Concentrations" = "FIC" umgerechnet (nach den Formeln

$$\frac{\text{MIC von A in Kombination mit B}}{\text{MIC von A allein}} = \text{FIC von A;}$$

$$\frac{\text{MIC von B in Kombination mit A}}{\text{MIC von B allein}} = \text{FIC von B;}$$

FIC von A + FIC von B = $\Sigma$ FIC), wobei eine $\Sigma$ FIC von $\leq$ 0.7 als Beweis für synergistische Interaktion der Kombinationspartner gilt.

Beispielsweise sind einige der gegen eine Reihe von Mikroorganismen gefundenen MIC-Werte ("minimum inhibitory concentrations") von Cefsulodin-Natrium, Mecillinam, Mezlocillin und Gentamicin allein, sowie die MIC-Werte einiger Mischungen bei denen Synergismus auftritt, in der Tabelle 1 aufgeführt.

3

## 0 004 925

TABELLE 1

| Mikroorganismus | MIC ($\mu$g/ml) | | | | |
|---|---|---|---|---|---|
| | Antibioti- cum A allein | Antibioti- cum A in Kombination mit B | Antibioti- cum B allein | Antibioti- cum B in Kombination mit A | $\Sigma$FIC |
| | Cefsulodin-Na (A) | | Mecillinam (B) | | |
| Pseudomonas aeruginosa 799A | 1 | 0.25 | 1000 | 32 | 0.28 |
| Escherichia coli 12—44 | 25 | 12.5 | 2 | 0.12 | 0.56 |
| Klebsiella pneumoniae 1132 | 25 | 6.2 | 1 | 0.25 | 0.50 |
| Klebsiella pneumoniae 1136 | 50 | 12.5 | 1 | 0.25 | 0.50 |
| Proteus rettgeri 1121 | 12.5 | 0.4 | 3.2 | 1.6 | 0.53 |
| | Cefsulodin-Na (A) | | Mezlocillin (B) | | |
| Pseudomonas aeruginosa G 121 | 1.2 | 0.04 | 25 | 12.5 | 0.53 |
| Staphylococcus aureus | 3.2 | 0.2 | 3.2 | 1.6 | 0.56 |
| Klebsiella pneumoniae 1 | 50 | 25 | 1.25 | 0.16 | 0.63 |
| Proteus mirabilis 1077 | 50 | 12.5 | 1 | 0.25 | 0.50 |
| | Cefsulodin-Na (A) | | Gentamicin (B) | | |
| Proteus rettgeri 1121 | 12.5 | 0.8 | 1 | 0.5 | 0.56 |
| Proteus aeruginosa 410 | 2 | 0.06 | 0.5; | 0.25 | 0.53 |
| Pseudomonas aeruginosa 1140 | 8 | 2 | 32 | 4 | 0.38 |
| Escherichia coli 576 | 64 | 32 | 2 | 0.25 | 0.63 |

2. Nachweis der synergistischen Wirkung *in vivo* bei Systeminfektionen an der Maus:

Die zu kombinierenden Substanzen A und B werden in 1:3 bis 1:3,3 Schritten so verdünnt, dass die mittlere Konzentration die der $ED_{50}$ entsprechende Menge von Substanz A bzw. B enthält.

Weibliche MF 2 Mäuse im Gewicht von 18—22 g werden zufällig in Gruppen (n=20) verteilt und mit einer standardisierten Keimsuspension, die je nach Stamm 5 bis > 20 $LD_{50}$ repräsentiert, intraperitoneal infiziert. Sofort nach der Infektion und 3 Stunden danach werden die Mäuse gruppenweise mit Verdünnungen der Substanzen A bzw. B, sowie mit deren Kombinationen subkutan behandelt. Diese Kombinationen werden kurz vor Applikation durch Mischung jeder Verdünnung der Substanz A mit jeder Verdünnung der Substanz B vorbereitet. Am 5. Tag nach der Infektion werden die Ueberlebensraten ermittelt. Die Ueberlebensraten der Mäuse, die mit den Substanzen A bzw. B alleine ermittelt wurden, werden mit denjenigen, die mit Kombinationen der Substanzen erhalten wurden, verglichen. Als synergistisch wirksam wird solche Kombination bezeichnet, die mehr Tiere vor dem Tod geschützt hat, als bei unabhängiger Wirkung der Substanzen zu erwarten wäre.

Die gefundenen Ueberlebensraten, ausgedrückt in Prozenten überlebender Tiere, bei Infektionen mit einigen reinen Mikroorganismen und bei Mischinfektionen, ist für Cefsulodin-Natrium, Mecillinam, Mezlocillin, Gentamicin und CGP 14 221/E allein, sowie wie für entsprechende Mischungen in Tabelle 2 angegeben.

**0 004 925**

TABELLE 2

| Infizierender Mikroorganismus | Antibioticum | Dosis s.c. mg/kg | % überlebende Tiere | |
|---|---|---|---|---|
| | | | Antibioticum allein | Kombination |
| Escherichia coli 205 | Cefsulodin-Na | 25 | 5 | 90 |
| | Mecillinam | 1 | 0 | |
| | Cefsulodin-Na | 20 | 5 | 75 |
| | Mecillinam | 2 | 0 | |
| | Cefsulodin-Na | 20 | 20 | 90 |
| | Mezlocillin | 50 | 0 | |
| | Cefsulodin-Na | 40 | 10 | 95 |
| | CGP 14221/E | 0.62 | 0 | |
| | Cefsulodin-Na | 20 | 0 | 90 |
| | CGP 14221/E | 2.5 | 0 | |
| Escherichia coli 205 $R^+_{TEM}$ | Cefsulodin-Na | 40 | 0 | 70 |
| | CGP 14221/E[1] | 1.2 | 10 | |
| Klebsiella pneumoniae 329 | Cefsulodin-Na | 40 | 0 | 85 |
| | Mecillinam | 300 | 0 | |
| | Cefsulodin-Na | 80 | 5 | 100 |
| | Mecillinam | 300 | 0 | |
| | Cefsulodin-Na | 40 | 0 | 90 |
| | Mezlocillin | 300 | 0 | |
| | Cefsulodin-Na | 50 | 0 | 85 |
| | Gentamicin | 0.2 | 30 | |
| Klebsiella pneumoniae 327 | Cefsulodin-Na | 100 | 0 | 85 |
| | CGP 14221/E | 20 | 0 | |
| | Cefsulodin-Na | 50 | 0 | 85 |
| | CGP 14221/E | 40 | 0 | |
| Klebsiella pneumoniae 329 + Escherichia coli 205 | Cefsulodin-Na | 100 | 0 | 70 |
| | CGP 14221/E | 20 | 0 | |

1) 7β-[2-(2-imino-1,3-thiazolin-4-yl)acetamido]-3-[1-(3-dimethylamino)-tetrazol-5-ylthio-methyl]-3-cephem-4-cartonsäure-hydrochlorid (Cefotiam)

3. Nachweis der synergistischen Wirkung *in vivo* bei experimenteller Pyelonephritis (Mischinfektionen) an der Maus:

Mäuse werden intravenös mit 10 mg/kg Caragheenan behandelt. Sieben Tage danach werden sie intravenös mit einer standardisierten Suspension von Bakterien infiziert. Eine Stunde nach der Infektion, 6 Stunden danach und jeweils zweimal täglich an 4 danach folgenden Tagen werden die Mäuse subkutan mit den zu untersuchenden Substanzen allein und deren Kombination behandelt. Zwei Tage nach der letzten Behandlung werden die Tiere getötet, seziert und auf Keimzahl in den Nieren untersucht.

Die gefundene Anzahl der Tiere, ausgedrückt in Prozenten, die bei Behandlung mit Cefsulodin-Natrium und Gentamicin allein und in Kombination völlige Befreiung (clearance) der Nieren von beiden Mikroorganismen zeigen, ist in Tabelle 3 angegeben.

5

TABELLE 3

| Infizierender Mikroorganismus | Antibioticum | Dosis s.c. mg/kg | Sefreiung der Nieren von beiden Mikroorganismen (% Tiere) | |
|---|---|---|---|---|
| | | | Antibioticum allein | Kombination |
| Pseudomonas aeruginosa + Escherichia coli | Cefsulodin-Na | 25 | 0 | 70 |
| | Gentamicin | 1 | 0 | |
| Pseudomonas aeruginosa + Klebsiella pneumoniae | Cefsulodin-Na | 25 | 10 | 90 |
| | Gentamicin | 2.5 | 40 | |
| | Cefsulodin-Na | 25 | 10 | 50 |
| | Gentamicin | 0.5 | 0 | |

Pharmazeutische Präparate

Beispiel 1

Trockenampullen oder Vials enthaltend 0,50 g Cefsuldin-Natrium und 0,02 g Mecillionam (1:0,04) werden wie folgt hergestellt:
Zusammensetzung (für 1000 Ampullen oder Vials):

| | |
|---|---|
| Cefsulodin-Natrium | 500 g |
| Mecillinam | 20 g |
| Mannit | 6 g |
| | 526 g |

Die Komponenten werden homogen gemischt und je 0.526 g der Mischung unter aseptischen Bedingungen in eine Ampulle oder ein Vial abgefüllt. Die Ampullen oder Vials werden verschlossen und geprüft.

Auf die gleiche Weise werden Ampullen oder Vials mit entsprechenden Mengen Mezlocillin, Gentamicin oder CGP 14221/E als zweiter Wirkstoffkomponente hergestellt.

Beispiel 2

Trockenampullen oder Vials enthaltend 1 g Cefsulodin-Natrium und 0,1 g Mecillinam (1:0,1) werden wie folgt hergestellt:
Zusammensetzung (für 1000 Ampullen oder Vials):

| | |
|---|---|
| Cefsulodin-Natrium | 1000 g |
| Mecillinam | 100 g |
| Mannit | 100 g |
| | 1200 g |

Die Komponenten werden homogen gemischt und je 1,2 g der Mischung unter aseptischen Bedingungen in eine Ampulle oder ein Vial abgefüllt. Die Ampullen oder Vials werden verschlossen und geprüft.

Auf die gleiche Weise werden Ampullen oder Vials mit entsprechenden Mengen Mezlocillin, Gentamicin oder CGP 14 221/E als zweiter Wirkstoffkomponente hergestellt.

Beispiel 3

Trockenampullen oder Vials enthaltend 2,0 g Cefsulodin-Natrium und 0,2 g Mecillinam (1:0,1) werden wie folgt hergestellt:
Zusammensetzung (für 1000 Ampullen oder Vials):

| | |
|---|---|
| Cefsulodin-Natrium | 2000 g |
| Mecillinam | 200 g |
| Mannit | 200 g |
| | 2400 g |

Die Komponenten werden homogen gemischt und je 2,4 g der Mischung unter aseptischen Bedingungen in eine Ampulle oder Vial abgefüllt. Die Ampullen oder Vials werden verschlossen und geprüft.

Auf die gleiche Weise werden Ampullen oder Vials mit entsprechenden Mengen Mezlocillin, Gentamicin oder CGP 14221/E als zweiter Wirkstoffkomponente hergestellt.

### Beispiel 4

Trockenampullen oder Vials enthaltend 2 g Cefsulodin-Natrium und 1 g Mecillinam (1:0,5 werden wie folgt hergestellt:

Zusammensetzung (für 1000 Ampullen oder Vials):

| | |
|---|---|
| Cefsulodin-Natrium | 2000 g |
| Mecillinam | 1000 g |
| Mannit | 300 g |
| | 3300 g |

Die Komponenten werden homogen gemischt und je 3,3 g der Mischung unter aseptischen Bedingungen in eine Ampulle oder Vials abgefüllt. Die Ampullen oder Vials werden verschlossen und geprüft.

Auf die gleiche Weise werden Ampullen oder Vials mit entsprechenden Mengen Mezlocillin, Gentamicin oder CGP 14 221/E als zweiter Wirkstoffkomponente hergestellt.

### Beispiel 5

Trockenampullen oder Vials enthaltend 1 g Cefsulodin-Natrium- und 1 g Mecillinam (1:1) werden wie folgt hergestellt:

Zusammensetzung (für 1000 Ampullen oder Vials):

| | |
|---|---|
| Cefsulodin-Natrium | 1000 g |
| Mecillinam | 1000 g |
| Mannit | 200 g |
| | 2200 g |

Die Komponenten werden homogen gemischt und je 2,2 g der Mischung unter aseptischen Bedingungen in eine Ampulle oder Vial abgefüllt. Die Ampullen oder Vials werden verschlossen und geprüft.

Auf die gleiche Weise werden Ampullen oder Vials mit entsprechenden Mengen Mezlocillin, Gentamicin oder CGP 14 221/E als zweiter Wirkstoff komponente hergestellt.

### Beispiel 6

Trockenampullen oder Vials enthaltend 1 g Cefsulodin-Natrium und 2,5 g Mecillinam (1:2,5) werden wie folgt hergestellt:

Zusammensetzung (für 1000 Ampullen oder Vials):

| | |
|---|---|
| Cefsulodin-Natrium | 1000 g |
| Mecillinam | 2500 g |
| Mannit | 350 g |
| | 3850 g |

Die Komponenten werden homogen gemischt und je 3,85 g der Mischung unter aseptischen Bedingungen in eine Ampulle oder Vial abgefüllt. Die Ampullen oder Vials werden verschlossen und geprüft.

Auf die gleiche Weise werden Ampullen oder Vials mit entsprechenden Mengen Mezlocillin oder CGP 14 221/E als zweiter Wirkstoffkomponente hergestellt.

### Beispiel 7

Trockenampullen oder Vials enthaltend 0,25 g Cefsulodin-Natrium und 0,937 g Mecillinam (1:3,75) werden wie folgt hergestellt:

Zusammensetzung (für 1000 Ampullen oder Vials):

| | |
|---|---|
| Cefsulodin-Natrium | 250 g |
| Mecillinam | 937 g |
| Mannit | 100 g |
| | 1287 g |

7

**0 004 925**

Die Komponenten werden homogen gemischt und je 1,287 g der Mischung unter aseptischen Bedingungen in eine Ampulle oder Vial abgefüllt. Die Ampullen oder Vials werden verschlossen und geprüft.

Auf die gleiche Weise werden Ampullen oder Vials mit entsprechenden Mengen Mezlocillin oder CGP 14 221/E als zweiter Wirkstoffkomponente hergestellt.

Beispiel 8

Trockenampullen oder Vials enthaltend 0,25 g Cefsulodin-Natrium und 1,875 g Mecillinam (1:7,5) werden wie folgt hergestellt:

Zusammensetzung (für 1000 Ampullen oder Vials):

| | |
|---|---|
| Cefsulodin-Natrium | 250 g |
| Mecillinam | 1875 g |
| Mannit | 200 g |
| | 2125 g |

Die Komponenten werden homogen gemischt und je 2.125 g der Mischung unter aseptischen Bedingungen in eine Ampulle oder Vial abgefüllt. Die Ampullen oder Vials werden verschlossen und geprüft.

Auf die gleiche Weise werden Ampullen oder Vials mit entsprechenden Mengen Mezlocillin oder CGP 14 221/E als zweiter Wirkstoffkomponente hergestellt.

Beispiel 9

Gemäss den Beispielen 1 bis 8 können unter Benutzung der gleichen Mischungsverhältnisse Trockenampullen oder Vials hergestellt werden, die neben Cefsulodin-Natrium und Mannit eine entsprechende Menge Clavulansäure, Penicillansäure-4,4-dioxid, Carbenicillin, Ticarcillin, Sulfocillin, Azlocillin, Bay K4999, Piperazillin, Cephacetril, Cefoxitin, Cefuroxim, Cefazolin, Cefamandol, CGP 11481, Cefotiam, SCE-1365, Cefotaxim, CGP-17845, Kanamycin A, Amikacin, Dibekacin, Tobramycin, Sisomycin, Netilmicin, SCH-22591, SCH-21420, oder ein pharmazeutisch annehmbares Salz davon, enthalten.

**Patentansprüche**

1. Synergistische antibiotische Mischungen bestehend aus Cefsulodin-Natrium und einem zweiten Antibiotikum aus der Gruppe Clavulansäure, Penicillansäure-4,4-dioxid, Azlocillin, Mezlocillin, Piperacillin, Mecillinam, Cephacetril, Cefoxitin, Cefotiam, Amikacin, Dibekacin, Tobramycin, Sisomicin oder Gentamicin oder einem Salz davon.

2. Synergistische Mischungen nach Patentanspruch 1, worin die Gewichtsverhältnisse Cefsulodin-Natrium zum zweiten Antibiotikum zwischen 1:0,001 und 1:4000 liegen.

3. Synergistische Mischungen nach einem der Patentansprüche 1 oder 2, bestehend aus Cefsulodin-Natrium und Clavulansäure oder einem Salz davon in einem Gewichtsverhältnis zwischen 1:0,001 und 1:4000.

4. Synergistische Mischungen nach einem der Patentansprüche 1 oder 2, bestehend aus Cefsulodin-Natrium und Penicillansäure-4,4-dioxid oder einem Salz davon in einem Gewichtsverhältnis zwischen 1:0,001 und 1:4000.

5. Synergistische Mischungen nach einem der Patentansprüche 1 oder 2, bestehend aus Cefsulodin-Natrium und Azlocillin oder einem Salz davon in einem Gewichtsverhältnis zwischen 1:0,04 und 1:3125.

6. Synergistische Mischungen nach einem der Patentansprüche 1 oder 2, bestehend aus Cefsulodin-Natrium und Mezlocillin oder einem Salz davon in einem Gewichtsverhältnis zwischen 1:0,04 und 1:3125.

7. Synergistische Mischungen nach einem der Patentansprüche 1 oder 2, bestehend aus Cefsulodin-Natrium und Piperacillin oder einem Salz davon in einem Gewichtsverhältnis zwischen 1:0,04 und 1:3125.

8. Synergistische Mischungen nach einem der Patentansprüche 1 oder 2, bestehend aus Cefsulodin-Natrium und Mecillinam oder einem Salz davon in einem Gewichtsverhältnis zwischen 1:0,04 und 1:128.

9. Synergistische Mischungen nach einem der Patentansprüche 1 oder 2, bestehend aus Cefsulodin-Natrium und Cephacetril oder einem Salz davon in einem Gewichtsverhältnis zwischen 1:0,0155 und 1:5.

10. Synergistische Mischungen nach einem der Patentansprüche 1 oder 2, bestehend aus

8

Cefsulodin-Natrium und Cefoxitin oder einem Salz davon in einem Gewichtsverhältnis zwischen 1:0,0155 und 1:5.

11. Synergistische Mischungen nach einem der Patentansprüche 1 oder 2, bestehend aus Cefsulodin-Natrium und Cefotiam oder einem Salz davon in einem Gewichtsverhältnis zwischen 1:0,0155 und 1:5.

12. Synergistische Mischungen nach einem der Patentansprüche 1 oder 2, bestehend aus Cefsulodin-Natrium und Amikacin oder einem Salz davon in einem Gewichtsverhältnis zwischen 1:0,004 und 1:5.

13. Synergistische Mischungen nach einem der Patentansprüche 1 oder 2, bestehend aus Cefsulodin-Natrium und Dibekacin oder einem Salz davon in einem Gewichtsverhältnis zwischen 1:0,004 und 1:5.

14. Synergistische Mischungen nach einem der Patentansprüche 1 oder 2, bestehend aus Cefsulodin-Natrium und Tobramycin oder einem Salz davon in einem Gewichtsverhältnis zwischen 1:0,004 und 1:5.

15. Synergistische Mischungen nach einem der Patentansprüche 1 oder 2, bestehend aus Cefsulodin-Natrium und Sisomicin oder einem Salz davon in einem Gewichtsverhältnis zwischen 1:0,004 und 1:5.

16. Synergistische Mischungen nach einem der Patentansprüche 1 oder 2, bestehend aus Cefsulodin-Natrium und Gentamicin oder einem Salz davon in einem Gewichtsverhältnis zwischen 1:0,004 und 1:5.

17. Pharmazeutische Präparate enthaltend eine der in den Patentansprüchen 1—16 genannten synergistischen Mischungen.

18. Verfahren zur Herstellung der pharmazeutischen Präparate von Patentanspruch 17, gekennzeichnet durch nichtchemische Massnahmen.

19. Synergistische Mischungen nach einem der Patentansprüche 1—17 zur Anwendung bei der Bekämpfung von Infektionen.

**Claims**

1. A synergistic mixture of antibiotics consisting of cefsulodin sodium and a second antibiotic selected from the group consisting of clavulanic acid, penicillanic acid 4,4-dioxide, azlocillin, mezlocillin, piperacillin, mecillinam, cephacetril, cefoxitin, cefotiam, amikacin, dibekacin, tobramycin, sisomicin or gentamicin, or a salt thereof.

2. A synergistic mixture according to claim 1, wherein the weight ratio of cefsulodin sodium to the second antibiotic is between 1:0.001 and 1:4000.

3. A synergistic mixture according to either of claims 1 or 2, consisting of cefsulodin sodium and clavulanic acid, or a salt thereof, in a weight ratio between 1:0.001 and 1:4000.

4. A synergistic mixture according to either of claims 1 or 2, consisting of cefsulodin sodium and penicillanic acid 4,4-dioxide, or a salt thereof, in a weight ratio between 1:0.001 and 1:4000.

5. A synergistic mixture according to either of claims 1 or 2, consisting of cefsulodin sodium and azlocillin, or a salt thereof, in a weight ratio between 1:0.04 and 1:3125.

6. A synergistic mixture according to either of claims 1 or 2, consisting of cefsulodin sodium and meziocillin, or a salt thereof, in a weight ratio between 1:0.04 and 1:3125.

7. A synergistic mixture according to either of claims 1 or 2, consisting of cefsulodin sodium and piperacillin, or a salt thereof, in a weight ratio between 1:0.04 and 1:3125.

8. A synergistic mixture according to either of claims 1 or 2, consisting of cefsulodin sodium and mecillinam, or a salt thereof, in a weight ratio between 1:0.04 and 1:128.

9. A synergistic mixture according to either of claims 1 or 2, consisting of cefsulodin sodium and cephacetril, or a salt thereof, in a weight ratio between 1:0.0155 and 1:5.

10. A synergistic mixture according to either of claims 1 or 2, consisting of cefsulodin sodium and cefoxitin, or a salt thereof, in a weight ratio between 1:0.0155 and 1:5.

11. A synergistic mixture according to either of claims 1 or 2, consisting of cefsulodin sodium and cefotiam, or a salt thereof, in a weight ratio between 1:0.0155 and 1:5.

12. A synergistic mixture according to either of claims 1 or 2, consisting of cefsulodin sodium and amikacin, or a salt thereof, in a weight ratio between 1:0.004 and 1:5.

13. A synergistic mixture according to either of claims 1 or 2, consisting of cefsulodin sodium and dibekacin, or a salt thereof, in a weight ratio between 1:0.004 and 1:5.

14. A synergistic mixture according to either of claims 1 or 2, consisting of cefsulodin sodium and tobramycin, or a salt thereof, in a weight ratio between 1:0.004 and 1:5.

15. A synergistic mixture according to either of claims 1 or 2, consisting of cefsulodin sodium and sisomicin, or a salt thereof, in a weight ratio between 1:0.004 and 1:5.

16. A synergistic mixture according to either of claims 1 or 2, consisting of cefsulodin sodium and gentamicin, or a salt thereof, in a weight ratio between 1:0.004 and 1:5.

17. A pharmaceutical preparation containing a synergistic mixture as claimed in any one of claims 1 to 16.

**0 004 925**

18. A process for the manufacture of the pharmaceutical preparation of claim 17 by non-chemical means.

19. Use of a synergistic mixture according to any one of claims 1 to 17 for combating infections.

## Revendications

1. Mélanges antibiotiques synergiques composés de cefsulodine sodique et d'un second antibiotique du groupe constitué par l'acide clavulanique, le 4,4-dioxyde de l'acide pénicillanique, l'azlocilline, la mezlocilline, la pipéracilline, le mécillinam, le céphacétril, la céfoxitine, le céfotiam, l'amikacine, la dibékacine, la tobramycine, la sisomicine ou la gentamicine ou un de leurs sels.

2. Mélanges synergiques selon la revendication 1, où les rapports pondéraux entre la cefsulodine sodique et le second antibiotique se situent entre 1:0,001 et 1:4000.

3. Mélanges synergiques selon l'une des revendications 1 ou 2, composés de cefsulodine sodique et d'acide clavulanique ou d'un de leurs sels dans un rapport pondéral compris entre 1:0,001 et 1:4000

4. Mélanges synergiques selon l'une des revendications 1 ou 2, composés de cefsulodine sodique et de 4,4-dioxyde de l'acide pénicillanique ou d'un de leurs sels dans un rapport pondéral compris entre 1:0,001 et 1:4000.

5. Mélanges synergiques selon l'une des revendications 1 ou 2, composés de cefsulodine sodique et d'azlocilline ou d'un de leurs sels dans un rapport pondéral compris entre 1:0,04 et 1:3125.

6. Mélanges synergiques selon l'une des revendications 1 ou 2, composé de cefsulodine sodique et de mezlocilline ou d'un de leurs sels dans un rapport pondéral compris entre 1:0,04 et 1:3125.

7. Mélanges synergiques selon l'une des revendications 1 ou 2, composés de cefsulodine sodique et de pipéracilline ou d'un de leurs sels dans un rapport pondéral compris entre 1:0,04 et 1:3125

8. Mélanges synergiques selon l'une des revendications 1 ou 2, composés de cefsulodine sodique et de mécillinam ou d'un de leurs sels dans un rapport pondéral compris entre 1:0,04 et 1:128.

9. Mélanges synergiques selon l'une des revendications 1 ou 2, composés de cefsulodine sodique et de céphacétril ou d'un de leurs sels dans un rapport pondéral compris entre 1:0,0155 et 1:5.

10. Mélanges synergiques selon l'une des revendications 1 ou 2, composés de cefsulodine sodique et de céfoxitine ou d'un de leurs sels dans un rapport pondéral compris entre 1:0,0155 et 1:5.

11. Mélanges synergiques selon l'une des revendications 1 ou 2, composés de cefsulodine sodique et de cefotiam ou d'un de leurs sels dans un rapport pondéral compris entre 1:0,0155 et 1:5.

12. Mélanges synergiques selon l'une des revendications 1 ou 2, composés de cefsulodine sodique et d'amikacine ou d'un de leurs sels dans un rapport pondéral compris entre 1:0,004 et 1:5.

13. Mélanges synergiques selon l'une des revendications 1 ou 2, composés de cefsulodine sodique et de dibékacine ou d'un de leurs sels dans un rapport pondéral compris entre 1:0,004 et 1:5.

14. Mélanges synergiques selon l'une des revendications 1 ou 2, composés de cefsulodine sodique et de tobramycine ou d'un de leurs sels dans un rapport pondéral compris entre 1:0,004 et 1:5.

15. Mélanges synergiques selon l'une des revendications 1 ou 2, composés de cefsulodine sodique et de sisomicine ou d'un de leurs sels dans un rapport pondéral compris entre 1:0,004 et 1:5.

16. Mélanges synergiques selon l'une des revendications 1 ou 2, composés de cefsulodine sodique et de gentamicine ou d'un de leurs sels dans un rapport pondéral compris entre 1:0,004 et 1:5.

17. Préparations pharmaceutiques comtenant l'un des mélanges synergiques mentionnés dans les revendications 1 à 16.

18. Procédé de préparation des préparations pharmaceutiques de la revendication 17, caractérisé par des mesures non chimiques.

19. Mélanges synergiques selon l'une des revendications 1 à 17 aux fins d'application dans la lutte contre les infections.